# EUROPEAN PATENT SPECIFICATION

(11) **EP 0 705 606 B1**
(45) Date of publication and mention of the grant of the patent: **18.03.1998**
(21) Application number: 95114673.7
(22) Date of filing: 18.09.1995
(51) Int. Cl.: A61K 31/54

(54) **Use of 5-Thia-1,4-diazabicyclo [4.2.0] octane-3,8-dioxo compounds in antitumor therapy**
Verwendung von 5-Thia-1,4-Diazabicyclo [4.2.0] Octane-3,8-dioxo Verbindungen in der Antitumor-Therapie
Utilisation de composés 5-thia-1,4-diazabicyclo [4.2.0] octane-3,8-dioxo dans la thérapie antitumorale

(30) Priority: 21.09.1994 HR 940577
(43) Date of publication of application: 10.04.1996
(73) Proprietor: PLIVA, farmaceutska, kemijska, prehrambena i kozmeticka industrija dionicko drustvo, 41000 Zagreb (HR)
(72) Inventor: Kovacevic, Mice, D. Sc., HR-41000 Zagreb (HR); Grdisa, Mira, D. Sc., HR-41040 Zagreb (HR); Pavelic, Kresimir, D. Sc., HR-41080 Zagreb (HR); Horvatic, Marjeta, M. Sc., HR-41000 Zagreb (HR); Mandic, Zora M. Sc., HR-41000 Zagreb (HR); Tomic, Mirjana M. Sc., HR-41000 Zagreb (HR); Herak, Jure J., D. Sc., HR-41000 Zagreb (HR); Lukic, Irena, M. Sc., HR-41000 Zagreb (HR)
(74) Representative: von Füner, Alexander, Dr.

(56) References cited:
- EP-A- 0 598 375
- TETRAHEDRON, vol. 49, no. 43, 1993 pages 9801-9814, Z. BRKIC ET AL. 'NEW FUNCTIONALIZED MONOCYCLIC beta-LACTAMS SUITABLE PRECURSORS FOR ANHYDRO 2-AZACEPHAMS'
- DATABASE WPI Week 8248 Derwent Publications Ltd., London, GB; AN 82-0356J & JP-A-57 171 994 (ASAHI CHEMICAL) , 22 October 1982
- DATABASE WPI Week 8036 Derwent Publications Ltd., London, GB; AN 80-63237C & JP-A-55 098 188 (MITSUI TOATSU CHEM INC) , 26 July 1980

## Description

EP 0 598 375 A2 of 25 May 1994 discloses 5-thia-1,4-diazabicyclo[4.2.0]octane-3,8-dioxo compounds of the general formula I, 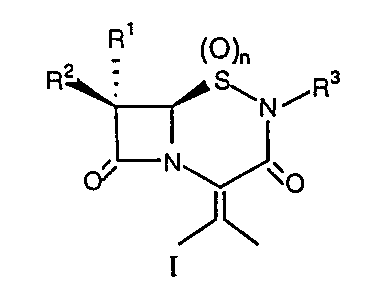 wherein the radicals have the following meanings:
- R¹: is hydrogen, halogen,
- R²: is hydrogen, halogen, amino, PhCH₂CONH, PhOCH₂CONH, phthalimido, o-MeNHCOC₆H₄CONH, 3-o-chlorophenyl-5-methylisoxazolyl-4-carbonyl amino,
- R³: is hydrogen, alkyl, benzyl, a heterocyclic ring e.g. isoxazole, pyrazole,
- n: is 1 or 2,
as well as their usefulness as active components of medicaments in antibacterial therapy.

The present invention refers to the use of compounds of the general formula I, wherein the radicals have the afore-mentioned meanings, for the preparation of a pharmaceutical for antitumor therapy.

It has not been known to use compounds of similar structures nor of any other β-lactam structures in antitumor therapy.

It was found that 5-thia-1,4-diazabicyclo[4.2.0]octane-3,8-dioxo analogs of the general formula I, wherein the radicals have the afore-mentioned meanings, strongly suppress the growth of the uterine cervix carcinoma cells (HeLa). By the use of compounds of formula I in concentrations from 10⁻³ to 10⁻⁶ M, a tumor-cell suppressive effect from 25 % to 65 % was established after 72 hours. The best effects were achieved at the concentration 5 x 10⁻⁴ M.

The following examples of the suppressive effect of the compounds of the general formula I upon the uterine cervix carcinoma indicate the possibility of the said compounds being also efficient in the suppression of other tumor cells.

### Testing process

Cells were cultured within a period of 12 hours in the growth medium DMEM (Dulbecco's Modified Eagle's Medium) supplemented with foetal calf serum (FCS) (10%), glutamine (2 mM), penicillin (100 U/ml), streptomycin (100 µg/ml) and Hepes (1 mM) at pH 7 at a temperature of 37°C under the flow of a mixture consisting of air and of 5% CO₂. The cells were seeded at a concentration of 10⁴ cells/ml on 24- or 96-well cell culture plates. After a period of 12 hours, solutions of compounds I in concentrations from 10⁻³ to 10⁻⁶ M were added. At the beginning all substances were prepared as 10⁻¹ M solutions in dimethylformamide and then the desired concentrations were obtained by diluting with the cell growth medium. 72 hours after the addition of the active substance the cells were counted by using an electronic counter and the suppressive effects of compounds I were determined as indicated in the following examples:

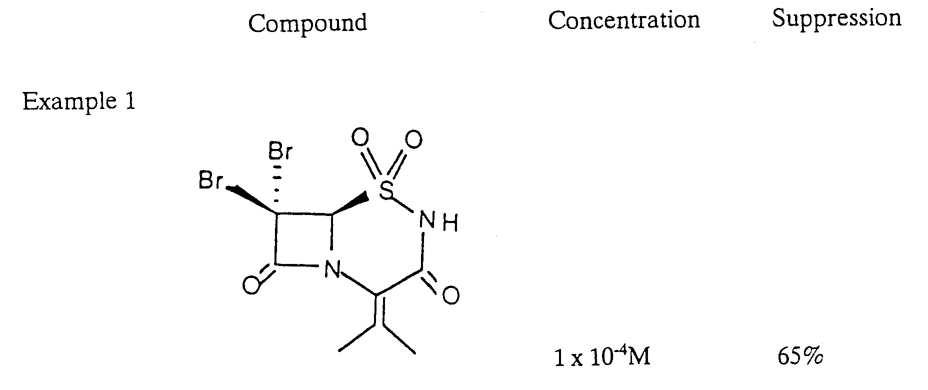

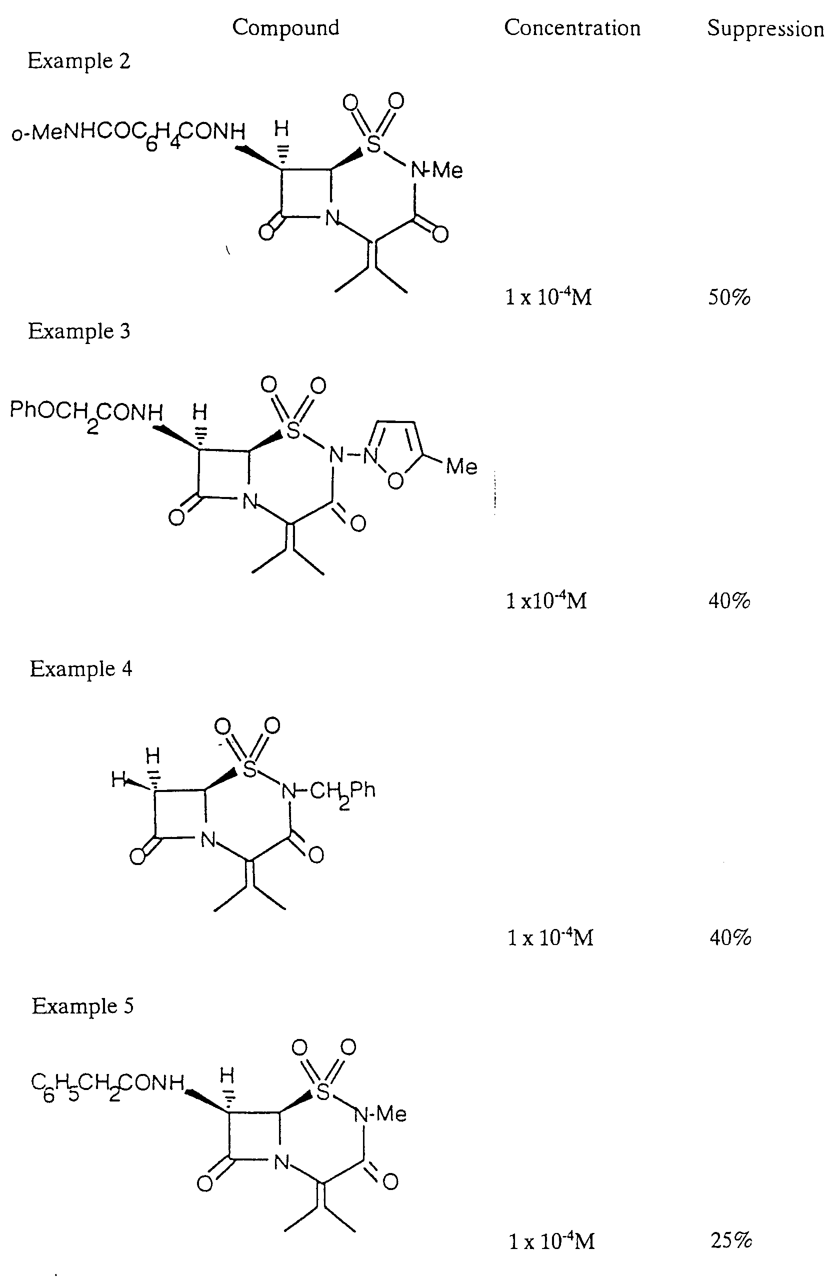

## Claims

1. The use of a 5-thia-1,4-diazabicyclo[4.2.0]octane-3,8-dioxo compound of the general formula I, 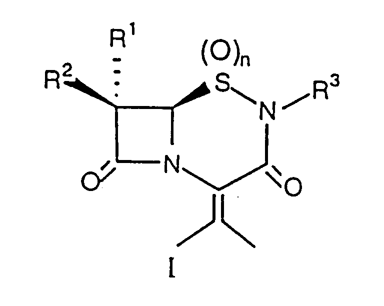 wherein the radicals have the following meanings:
R¹ is hydrogen, halogen,
R² is hydrogen, halogen, amino, PhCH₂CONH, PhOCH₂CONH, phthalimido, o-MeNHCOC₆H₄CONH, 3-o-chlorophenyl-5-methylisoxazolyl-4-carbonyl amino,
R³ is hydrogen, alkyl, benzyl, a heterocyclic ring e.g. isoxazole, pyrazole,
n is 1 or 2,
for the preparation of a pharmaceutical for antitumor therapy.

## Patentansprüche

1. Verwendung einer 5-Thia-1,4-diazabicyclo [4.2.0] octan-3,8-dioxo-Verbindung der allgemeinen Formel (I) 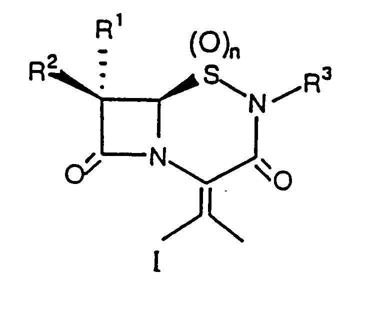 worin die Radikale die folgenden Bedeutungen besitzen:
R¹ ist Wasserstoff, Halogen,
R² ist Wasserstoff, Halogen, Amino, PhCH₂CONH, PhOCH₂CONH, Phthalimido, o-MeNHCOC₆H₄CONH, 3-o-Chlorphenyl-5-methylisoxazolyl-4-carbonylamino,
R³ ist Wasserstoff, Alkyl, Benzyl, ein heterocyclischer Ring z.B. Isoxazol, Pyrazol,
n ist 1 oder 2,
zur Herstellung von Arzneimitteln für Antitumor-Therapie.

## Revendications

1. Utilisation d'un composé du type 5-thia-1,4-diazabicyclo[4.2.0]octane-3,8-dioxo de la formule générale I, 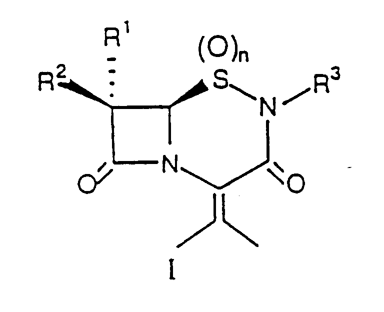 dans laquelle les radicaux ont les significations suivantes :
R¹ est un radical hydrogène, halogène,
R² est un radical hydrogène, halogène, amino, PhCH₂CONH, PhOCH₂CONH, phtalimido, o-MeNHCOC₆H₄CONH, 3-o-chlo rophényl-5-méthylisoxazolyl-4-carbonylamino,
R³ est un radical hydrogène, alkyle, benzyle, un noyau hétérocyclique, par exemple, isoxazole, pyrazole,
n est égal à 1 ou à 2,
pour la préparation d'un produit pharmaceutique destinée à la thérapie antitumorale.
